(19) Europäisches Patentamt
European Patent Office
Office européen des brevets

(11) Veröffentlichungsnummer: **0 270 906**
**B1**

(12) # EUROPÄISCHE PATENTSCHRIFT

(45) Veröffentlichungstag der Patentschrift: **21.02.90**

(51) Int. Cl.⁴: **C07C 265/12**, C07C 265/14, C07C 263/10, C08G 18/77

(21) Anmeldenummer: **87117283.9**

(22) Anmeldetag: **24.11.87**

(54) **Diisocyanate und Diisocyanatgemische, ein Verfahren zu ihrer Herstellung und ihrer Verwendung bei der Herstellung von Polyurethankunststoffen.**

(30) Priorität: **06.12.86 DE 3641702**

(43) Veröffentlichungstag der Anmeldung:
**15.06.88 Patentblatt 88/24**

(45) Bekanntmachung des Hinweises auf die Patenterteilung:
**21.02.90 Patentblatt 90/8**

(84) Benannte Vertragsstaaten:
**BE DE ES FR GB IT NL SE**

(56) Entgegenhaltungen:
**EP-A- 0 183 115**

**CHEMICAL ABSTRACTS, Band 49, Nr. 1, 10 Januar 1955, Columbus, Ohio, USA F.H. MC MILLIAN "Diaryloxyalkane derivatives. Some miscellaneous diphenoxypropanes" Spalte 214, Zusammenfassung a-d**

(73) Patentinhaber: **BAYER AG,
D-5090 Leverkusen 1 Bayerwerk(DE)**

(72) Erfinder: **König, Klaus, Dr., Zum Hahnenberg 40,
D-5068 Odenthal(DE)**
Erfinder: **Heitkämper, Peter, Dr., Fliederweg 14,
D-4047 Dormagen 11(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft α-(4-Isocyanatophenoxy-ω-(4-iso-cyanato-3-methyl-phenoxy)-alkane, deren Gemische mit den entsprechenden, Methylsubstituenten-freien und den entsprechenden symmetrisch zweifach methylsubstituierten Diisocyanaten, ein Verfahren zur Herstellung von derartigen Diisocyanaten bzw. Diisocyanatgemischen durch Umsetzung der entsprechenden Diamine bzw. Diamingemische oder ihrer Addukte mit Chlorwasserstoff oder Kohlendioxid mit Phosgen, sowie ihre Verwendung als Aufbaukomponente bei der Herstellung von Polyurethankunststoffen.

Die Eigenschaften von Polyurethankunststoffen, insbesondere von Polyurethanelastomeren hängen u.a. wesentlich von der Natur des bei der Herstellung der Kunststoffe eingesetzten Polyisocyanats ab. Besonders hochwertige Polyurethanelastomere werden so beispielsweise bei Verwendung von 1,5-Diisocyanatonaphthalin als Diisocyanatkomponente erhalten. Insbesondere zeichnen sich Gießelastomere auf Basis dieses Diisocyanats durch hervorragende mechanische Eigenschaften aus (vgl. z.B. Becker, Braun, Kunststoff-Handbuch, Band 7, 2. Auflage (1983), Carl-Hanser-Verlag). 1,5-Diisocyanatonaphthalin ist jedoch mit dem Nachteil behaftet, daß der zu seiner Herstellung eingesetzte Grundrohstoff, Naphthalin, nur in beschränkten Mengen zur Verfügung steht. Die Nitrierung von Naphthalin führt außerdem zwangsläufig zu einem Isomerengemisch von Nitronaphthalinen, aus dem 1,5-Dinitronaphthalin isoliert werden muß. Die destillative Reinigung des 1,5-Diisocyanatonaphthalins, welches aus der Dinitroverbindung durch Hydrierung und anschließende Phosgenierung des resultierenden Diamins erhalten wird, bereitet ebenfalls Schwierigkeiten, da es zur Sublimation neigt. Dies alles führt dazu, daß 1,5-Diisocyanatonaphthalin mit hohen Kosten belastet ist.

Auch die Verarbeitung von 1,5-Diisocyanatonaphthalin ist häufig problematisch, weil sein Schmelzpunkt und sein Dampfdruck relativ hoch sind. Diese Eigenschaften erlauben es oft nicht, 1,5-Diisocyanatonaphthalin ohne weiteres als Schmelze umzusetzen. Es sind dann technisch aufwendige Verarbeitungsmethoden und Schutzmaßnahmen erforderlich, um chemische und arbeitsmedizinische Schwierigkeiten zu vermeiden.

Es hat daher nicht an Versuchen gefehlt, für 1,5-Diisocyanatonaphthalin als Diisocyanatkomponente bei der Herstellung hochwertiger Polyurethankunststoffe einen gleichwertigen Ersatz zu finden.

So ist beispielsweise in DE-OS 3 138 421 und DE-OS 3 138 422 die Herstellung von Polyurethan-Elastomeren unter Verwendung von 4,4'-Diisocyanato-diphenylethan-(1,2) als Diisocyanat-Komponente beschrieben. Zwar können mit diesem Diisocyanat Kunststoffe mit guten mechanischen Eigenschaften erhalten werden; jedoch ist die Herstellung von 4,4'-Diisocyanato-diphenylethan-(1,2) sehr umständlich und aufwendig und bis jetzt auch technisch schwer realisierbar.

Weiterhin sind zahlreiche Versuche unternommen worden, das vergleichsweise kostengünstige 4,4'-Diisocyanato-diphenylmethan anstelle von 1,5-Diisocyanatonaphthalin zur Herstellung von hochwertigen Polyurethanelastomeren zu verwenden, jedoch sind bislang alle Versuche gescheitert, auf Basis dieses Diisocyanats Polyurethanelastomere herzustellen, die bezüglich ihrer mechanischen und thermischen Eigenschaften den Polyurethanelastomeren auf Basis von 1,5-Diisocyanatonaphthalin entsprechen.

Als Aufbaukomponenten für Polyurethankunststoffe sind in der DE-OS 3 442 689 α,ω-Bis-(4-isocyanato-phenoxy)-ethan, -n-butan und -n-hexan beschrieben. Der Einsatz dieser Diisocyanate in Polyurethan-Elastomeren liefert zwar Produkte mit hervorragenden Eigenschaften, gegebenenfalls ist jedoch die Verarbeitung dieser Isocyanate mit Schwierigkeiten verbunden: Einerseits ergeben diese Diisocyanate, wie gewünscht, Umsetzungsprodukte mit hochkristallinen Hartsegmenten; andererseits besitzen diese Diisocyanate eine große Reaktivität. Die Folge kann sein, daß sich bei ihrer Umsetzung mit Polyolen spontan Feststoffe aus den flüssigen Reaktionsgemischen abscheiden. Derartige Ausfällungen stören aber den technischen Prozeß solcher Umsetzungen, insbesondere die Herstellung von Präpolymeren.

Es bestand somit ein Interesse an Diisocyanaten mit gleicher Grundstruktur, jedoch verringerter Reaktivität. Ein erster Schritt in dieser Richtung kann in den Diisocyanaten gemäß DE-OS 3 525 606 (vgl. auch EP-A-0 183 115) gesehen werden. Die in dieser Veröffentlichung beschriebenen α,ω-Bis-(4-isocyanato-3-methyl-phenoxy)-alkane bereiten bezüglich der Umsetzung mit Polyolen keine Schwierigkeiten durch Feststoffausfällungen, und die erhaltenen Polyurethankunststoffe besitzen ebenfalls ein hervorragendes Werteniveau. Nachteilig ist jedoch, daß die Reaktivität dieser Diisocyanate durch die zwei Methylsubstituenten unerwartet stark verringert ist, so daß die Umsetzung der Isocyanate mit Polyolen bei den üblichen Reaktionstemperaturen und -zeiten im allgemeinen einen Zusatz von Katalysatoren erfordert. Das aber kann, insbesondere bei der Herstellung von Präpolymeren, ungünstig sein.

Es war daher die der Erfindung zugrunge liegende Aufgabe, neue Diisocyanate bzw. Diisocyanatgemische zur Verfügung zu stellen, welche bezüglich ihrer Eignung zur Herstellung von hochwertigen Polyurethanelastomeren dem 1,5-Diisocyanatonaphthalin vergleichbar und nicht mit den Nachteilen der genannten Diisocyanate des Standes der Technik behaftet sind.

Diese Aufgabe konnte mit der Bereitstellung der nachstehend näher beschriebenen erfindungsgemäßen Diisocyanate bzw. Diisocyanatgemische bzw. dem Verfahren zu ihrer Herstellung gelöst werden.

Gegenstand der Erfindung sind Diisocyanate oder Diisocyanatgemische, dadurch gekennzeichnet, daß sie

a) zu 30 bis 100 Gew.-% aus Diisocyanaten der allgemeinen Formel

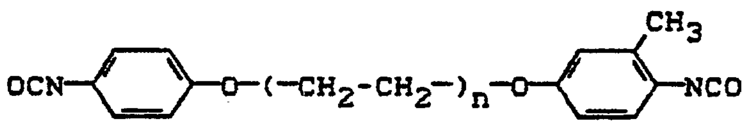 ,

b) zu 0 bis 50 Gew.-% aus Diisocyanaten der allgemeinen Formel

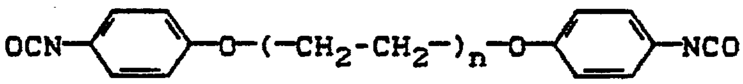

und
c) zu 0 bis 50 Gew.-% aus Diisocyanaten der allgemeinen Formel

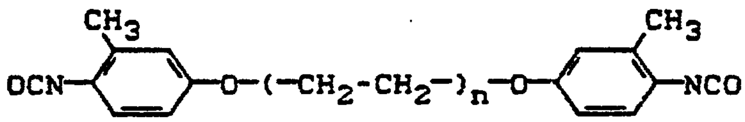

bestehen, wobei
n in diesen Formeln die Bedeutung von 1, 2 oder 3 hat,

und wobei sich die genannten Prozentsätze zu 100 ergänzen.

Diese erfindungsgemäßen Diisocyanate bzw. Diisocyanatgemische sind nicht nur bezüglich ihrer Verarbeitbarkeit in Diisocyanaten gemäß DE-OS 3 442 689 bzw. EP-A-0 183 115 überlegen, sondern eignen sich auch weit besser zur Herstellung von hochwertigen Polyurethanelastomeren als das in "Annalen der Chemie", 562 (1949), auf Seite 129 beschriebene 1,2-Bis-(2-Isocyanatophenoxy)-ethan bzw. das in "Journal of the American Chemical Society", 74 (1952), Seiten 5230 beschriebene 1,3-Bis-(4-isocyanatophenoxy)-propan.

Gegenstand der Erfindung ist auch ein Verfahren zur Herstellung der erfindungsgemäßen Diisocyanate bzw. Diisocyanatgemische, dadurch gekennzeichnet, daß man

a) Diamine der allgemeinen Formel

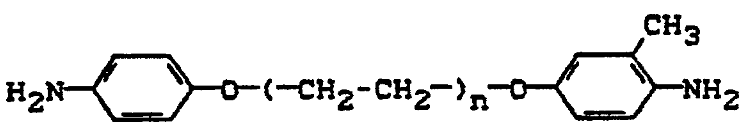

oder Gemische von diesen Diaminen mit
b) Diaminen der allgemeinen Formel

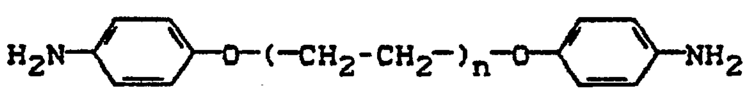

und mit
c) Diaminen der allgemeinen Formel

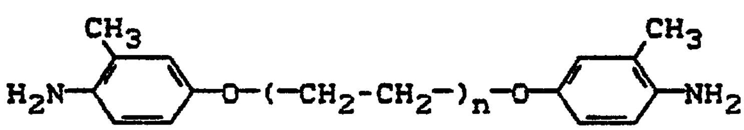

oder deren Addukte mit Chlorwasserstoff oder Kohlendioxid in an sich bekannter Weise mit Phosgen umsetzt, wobei n in diesen Formeln die obengenannte Bedeutung hat, und wobei die mengenmäßige Zusammensetzung der gegebenenfalls eingesetzten Diamingemische so gewählt wird, daß Diisocyanatgemische der erfindungsgemäßen Zusammensetzung resultieren.

Gegenstand der Erfindung ist auch die Verwendung der erfindungsgemäßen Diisocyanate bzw. Diisocyanatgemische als Aufbaukomponente bei der Herstellung von Polyurethankunststoffen nach dem Isocyanat-Polyadditionsverfahren.

Die beim erfindungsgemäßen Verfahren einzusetzenden Diamine der allgemeinen Formel

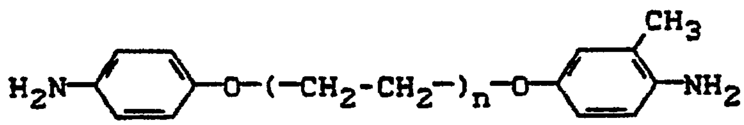

können vorteilhaft aus den entsprechenden α-(4-Nitrophenoxy)-ω-(4-nitro-3-methyl-phenoxy)-alkanen durch Reduktion mit unedlen Metallen, z.B. Zinn oder Eisen, in Gegenwart von Säuren oder durch katalytische Hydrierung hergestellt werden. Selbstverständlich können die Diamine auch nach jeder beliebigen anderen Methode hergestellt werden.

Die den Diaminen zugrunde ligenden Dinitroverbindungen können beispielsweise durch zweistufige Kondensation gemäß dem Stand der Technik (z.B. in "The Chemistry of the Ether Linkage", Herausgeber S. Patai, Interscience Publisher, 1967, Seiten 445 bis 498) hergestellt werden. Zur Verdeutlichung sollen im folgenden beispielhaft einige Synthesemöglichkeiten für 1-(4-Nitrophenoxy)-2-(4-nitro-3-methylphenoxy)-ethan beschrieben werden:

Durch Kondensation von 2-Chlorethanol mit Alkali-4-nitrophenolat wird in der ersten Stufe 2-(4-Nitrophenoxy)-ethanol gewonnen, das auch auf anderem Wege durch Umsetzung von 4-Nitrophenol mit Ethylenoxid oder mit Glykolcarbonat zugänglich ist (vgl. Houben-Weyl, Methoden der organischen Chemie, Band 6/3, Georg Thieme Verlag, 1965, Seite 75). 2-(4-Nitrophenoxy)-ethanol kann dann in der zweiten Stufe mit 2-Nitro-5-chlortoluol in Gegenwart von Basen zum gewünschten Produkt umgesetzt werden. Eine weitere Synthesemöglichkeit besteht darin, 2-(4-Nitrophenoxy)-ethanol mit 4-Toluolsulfonsäurechlorid zum entsprechenden Sulfonsäureester umzusetzen, der dann durch Reaktion mit 4-Nitro-3-methyl-phenol zur gesuchten Dinitroverbindung führt (vgl. Houben-Weyl, Methoden der organischen Chemie, Band 6/3, Georg Thieme Verlag, 1965, Seiten 68 und 69).

Weiterhin kann auch durch Umkehrung der Reihenfolge in der ersten Stufe 2-(4-Nitro-3-methyl-phenoxy)-ethanol hergestellt werden, aus dem dann mit 4-Nitrochlorbenzol oder, nach Veresterung mit 4-Toluolsulfonsäurechlorid, mit 4-Nitrophenol die gewünschte Verbindung gewonnen werden kann.

Die den zur erfindungsgemäßen Umsetzung einzusetzenden Diaminen zugrunde liegenden Dinitroverbindungen können auch beispielsweise durch Kondensation von α,ω-Dihalogenalkanen ("Halogen" steht hier und nachstehend für Chlor oder Brom) mit Gemischen von Alkali-4-nitrophenolat und Alkali-4-nitro-3-methyl-phenolat oder durch Kondensatin von α,ω-Alkandiolen mit Gemischen von 4-Nitro-halogenbenzol und 2-Nitro-5-halogen-toluol in Gegenwart von Basen hergestellt werden. Dabei entstehen in Abhängigkeit vom eingesetzten Produktverhältnis statistische Mischungen, die neben den α-(4-Nitrophenoxy)-ω-(4-nitro-3-methyl-phenoxy)-alkanen auch Bis-(4-nitrophenoxy)-alkane und Bis-(4-nitro-3-methyl-phenoxy)-alkane enthalten. Aus den hierbei anfallenden Gemischen können gewünschtenfalls die reinen, den Diaminen der zuletzt genannten Formel entsprechenden Dinitroverbindungen isoliert werden, beispielsweise durch fraktionierte Kristallisation, es wäre jedoch auch möglich, die reinen Diamine der zuletzt genannten allgemeinen Formel nach der Hydrierung des Gemischs der Dinitroverbindungen aus dem resultierenden Diamingemisch durch Destillation zu isolieren. Auch eine destillative Reindarstellung der Komponente a) der erfindungsgemäßen Gemische auf der Diisocyanatstufe wäre im Prinzip möglich.

Besonders vorteilhaft werden jedoch die Gemische von Dinitroverbindungen ohne weitere Auftrennung in Einzelkomponenten zu den entsprechenden Diamingemischen und diese zu den erfindungsgemäßen Diisocyanatgemischen weiterverarbeitet.

Auf diese Weise sind besonders interessante Diisocyanatgemische zugänglich, die sich

a) zumindest zu 30 Gew.-%, vorzugsweise zu 30 bis 50 Gew.-% und besonders bevorzugt zu 35 bis 50 Gew.-% aus Diisocyanaten der allgemeinen Formel

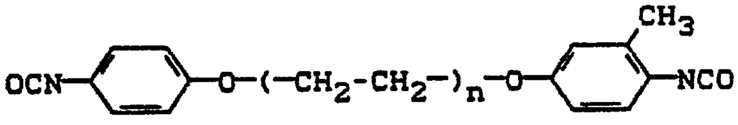

,

b) zu bis zu 50 Gew.-%, vorzugsweise 5 bis 45 Gew.-% und besonders bevorzugt zu 10 bis 35 Gew.-% aus Diisocyanaten der allgemeinen Formel

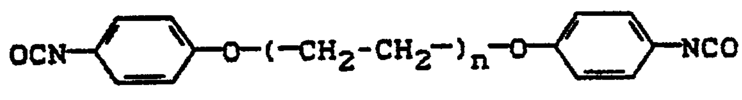

und

c) zu bis zu 50 Gew.-%, vorzugsweise 5 bis 45 Gew.-% und besonders bevorzugt zu 10 bis 35 Gew.-% aus Diisocyanaten der allgemeinen Formel

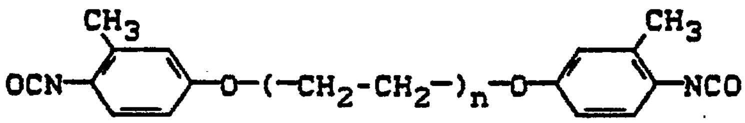

zusammensetzen, wobei sich die genannten Prozentsätze jeweils zu 100 ergänzen. In diesen Gemischen kann auch eine "asymmetrische" Verteilung innerhalb der genannten Bereich vorliegen, falls beispielsweise entsprechend unterschiedliche Mengen an 4-Nitro-halogenbenzol und 2-Nitro-5-halogentoluol zur Herstellung der Dinitroverbindungen eingesetzt werden. Die mengenmäßige Zusammensetzung der erfindungsgemäßen Diisocyanatgemische entspricht in erster Näherung der mengenmäßigen Zusammensetzung der ihnen zugrunde liegenden Diamingemische bzw. der diesen Diamingemischen zugrunde liegenden Gemische der Dinitroverbindungen. Die Zusammensetzung der erfindungsgemäßen Diisocyanatgemische kann daher auf einfache Weise durch geeignete Wahl der Mengenverhältnisse der zuletzt genannten Mononitroverbindungen eingestellt werden.

Die erfindungsgemäß zu phosgenierenden Diamine bzw. Diamin-Gemische können beim erfindungsgemäßen Verfahren in technischer Reinheit, wie sie bei ihrer Herstellung anfallen, oder auch in gereinigter Form zum Einsatz gelangen. Die Reinigung kann beispielsweise durch Lösen in Dimethylformamid und anschließendes Ausfällen mit Wasser oder destillativ erfolgen.

Beim erfindungsgemäßen Verfahren können die Diamine bzw. die Diamin-Gemische als solche oder auch in Form ihrer Additionsverbindungen mit Chlorwasserstoff oder mit Kohlendioxid zum Einsatz gelangen. Die erfindungsgemäße Phosgenierung erfolgt im übrigen nach an sich bekannten Methoden, wie sie beispielsweise in Liebigs Annalgen der Chemie, Band 562, Jahrgang 1949, Seiten 75 bis 109, in Ullmanns Encyclopädie der technischen Chemie, Band 14, 4. Auflage, 1977, Seiten 350 bis 354 oder in Houben-Weyl, Methoden der organischen Chemie, Band E4, 4. Auflage, 1983, Seiten 741 bis 753 beschrieben sind.

Die Umsetzung kann kontinuierlich oder diskontinuierlich, bevorzugt in Gegenwart eines inerten Lösungsmittels, durchgeführt werden. Geeignete Lösungsmittel sind die für Phosgenierungen üblicherweise verwendeten Lösungsmittel, wie aliphatische, cycloaliphatische oder aromatische Kohlenwasserstoffe, Halogenkohlenwasserstoffe, Nitrokohlenwasserstoffe, aliphatisch-aromatische Ether, aromatische Ether, Carbonsäureester, Carbonsäurenitrile, Sulfone, Phosphorsäurehalogenide oder Phosphorsäureester. Als Beispiele für geeignete Lösungsmittel seien genannte: Trimethylpentan, Decahydronaphthalin, Toluol, 1,2-Dichlorethan, Chlorbenzol, Chlortoluol, 1,2-Dichlorbenzol, Nitrobenzol, Anisol, Phenetol, Diphenylether, Essigsäurebutylester, Tetramethylensulfon, Phosphoroxychlorid, Phosphorsäuretrimethylester. Bevorzugt wird technisches Chlorbenzol oder technisches 1,2-Dichlorbenzol als Lösungsmittel verwendet. Beliebige Gemische der beispielhaft genannten Lösungsmittel können selbstverständlich ebenfalls eingesetzt werden. In den meisten der beispielhaft genannten Lösungsmittel weisen die erfindungsgemäßen Diisocyanate bei niedrigen Temperaturen nur eine geringe Löslichkeit auf.

Bei der Durchführung des erfindungsgemäßen Verfahrens erfüllen demzufolge die beispielhaft genannten Lösungsmittel, insbesondere bei den niedrigen Phosgeniertemperaturen, vor allem die Funktion eines Suspendiermittels für das Diamin bzw. dessen Chlorwasserstoff- oder Kohlendioxid-Addukte und erst bei höheren Temperaturen und mit zunehmender Überführung des Ausgangsmaterials in das Diisocyanat die Funktion eines echten Lösungsmittels für das Ausgangsmaterial und insbesondere das Verfahrensprodukt.

Bei den beim erfindungsgemäßen Verfahren einzusetzenden Gemischen aus zu phosgenierendem Ausgangsmaterial und Lösungsmittel handelt es sich im allgemeinen um "Lösungssuspensionen" mit einem Gehalt an Diamin bzw. Diaminaddukt der oben beispielhaft genannten Art von ca. 2 bis 70 Gew.-%. Der Begriff "Lösungssuspension" soll andeuten, daß die Ausgangsmaterialien, insbesondere im Falle der bevorzugten Verwendung der Diamine, teilweise gelöst und teilweise suspendiert vorliegen.

Die erfindungsgemäße Phosgenierungsreaktion kann beispielsweise nach dem bekannten Prinzip der "Kalt-Heiß-Phosgenierung" zweistufig oder nach dem Prinzip der "Heiß-Phosgenierung" einstufig erfolgen. Bei der "Kalt- Heiß-Phosgenierung" erfolgt die Umsetzung des zu phosgenierenden Ausgangsmaterials zu Beginn der Reaktion im allgemeinen bei -20 bis +40°C, vorzugsweise -10 bis +30°C und die anschließende Heißphosgenierung bei 40 bis 260°C, vorzugsweise 80 bis 220°C. Bei dieser "Kalt-Heiß-Phosgenierung" kann der Bereich zwischen der Anfangstemperatur und der erhöhten Temperatur gleichmäßig oder in Sprüngen durchlaufen werden.

Bei der "Heißphosgenierung" kommt das zu phosgenierende Ausgangsmaterial mit dem Phosgen sofort bei Temperaturen von 40 bis 260°C, vorzugsweise 80 bis 220°C, in Kontakt.

Die genannte "Kalt-Heiß-Phosgenierung" ist die bevorzugte Verfahrensweise im Falle der Phosgenierung des Diamins (anstelle der Chlorwasserstoff- bzw. Kohlendioxid-Addukte). Hierbei erfolgt bei den genannten tiefen Temperaturen keine nennenswerte Umsetzung zwischen dem suspendierten Diamin und dem zugefügten Phosgen. Erst bei der nachfolgenden Erhöhung der Temperatur beginnt das Diamin

mit dem Phosgen entsprechend seiner zunehmenden Löslichkeit zu reagieren.

Bei allen Varianten der erfindungsgemäßen Phosgenierung erfolgt diese vorzugsweise unter Normaldruck oder bei erhöhtem Druck. Der Reaktionsdruck liegt im allgemeinen bei 0.9 bis 100, vorzugsweise 1 bis 60 bar.

Im allgemeinen wird bei der erfindungsgemäßen Phosgenierungsreaktion das zu phosgenierende Ausgangsmaterial mit einer 1- bis 10-fachen, vorzugsweise 1,05- bis 6-fachen stöchiometrischen Menge an Phosgen zusammengebracht. Die genannte Phosgenmenge kann in einer Portion oder auch in Teilmengen in das Reaktionsgemisch eingegeben werden. Es kann vorteilhaft sein, z.B. bei einer diskontinuierlichen Arbeitsweise, erst einen Teil der zu verwendenden Phosgenmenge in das Reaktionsgemisch einzubringen und den restlichen Anteil in weiteren Portionen oder stetig über einen längeren Zeitraum verteilt in das Reaktionsgemisch einzubringen.

Grundsätzlich läßt sich die erfindungsgemäße Phosgenierung der Diamine durch Zugabe von Katalysatoren, beispielsweise Dimethylformamid, und/oder Säureakzeptoren, beispielsweise Pyridin, beschleunigen. Im allgemeinen jedoch sind die Reaktionsgeschwindigkeiten bei der Phosgenierung des Diamins auch ohne Zugabe eines derartigen Katalysators ausreichend.

Die Reaktionsdauer bei der erfindungsgemäßen Phosgenierung ist von den angewandten Reaktionsbedingungen, insbesondere von den Reaktionstemperaturen, vom Phosgen-Überschuß, von der Verdünnung mit Lösungsmittel und von gegebenenfalls zugegebenen Katalysatoren und/oder Säureakzeptoren abhängig.

Nach Beendigung der Phosgenierungsreaktion wird das Reaktionsgemisch in an sich bekannter Weise durch Abtrennung von gasförmigen Bestandteilen (Chlorwasserstoff, überschüssiges Phosgen) und destillativer Entfernung des Lösungsmittels aufgearbeitet. Vor der destillativen Entfernung des Lösungsmittels können gegebenenfalls vorliegende feste Nebenprodukte durch Filtration oder Zentrifugieren entfernt werden. Das nach der destillativen Entfernung des Lösungsmittels als Destillationsrückstand anfallende Rohprodukt kann gewünschtenfalls durch Umkristallisieren aus einem geeigneten inerten Lösungsmittel, beispielsweise Toluol oder vorzugsweise destillativ gereinigt werden.

Obwohl es sich bei den erfindungsgemäßen Diisocyanaten um thermostabile Substanzen handelt, kann es zweckmäßig sein, die Reindestillation der Diisocyanate ohne große Temperaturbelastung, beispielsweise mittels eines Dünnschichtverdampfers durchzuführen. Gewünschtenfalls können die erfindungsgemäßen Diisocyanate nach ihrer Reindarstellung auch durch Tempern bei Temperaturen von 160 bis 250°C, vorzugsweise 180 bis 230°C, von störenden Nebenprodukten, beispielsweise thermolabilen, chlorhaltigen Verbindungen befreit werden.

Bei der erfindungsgemäßen Verwendung der neuen Diisocyanate bzw. Diisocyanatgemische zur Herstellung von Polyurethankunststoffen, insbesondere von massiven oder zellförmigen Polyurethanelastomeren werden die erfindungsgemäßen Diisocyanate bzw. Diisocyanatgemische anstelle der bisland für diesen Verwendungszweck eingesetzten Diisocyanate mit den bekannten Reaktionspartnern zur Umsetzung gebracht (vgl. diesbezüglich beispielsweise die bereits eingangs genannten Literaturstellen oder auch "Kunststoff-Handbuch", Band VII, "Polyurethane" von Vieweg und Höchtlen, Carl Hanser Verlag München (1966), insbesondere Seiten 206-297).

Anstelle der Verwendung von erfindungsgemäßen Diisocyanatgemischen, wie sie beim erfindungsgemäßen Verfahren erhalten werden können, können bei der erfindungsgemäßen Verwendung auch entsprechende Gemische eingesetzt werden, die durch Abmischung eines vorab in reinem Zustand hergestellten, einfach Methyl-substituierten Diisocyanat mit separat hergestellten keinen oder zwei Methylsubstituenten aufweisenden Diisocyanaten erhalten worden sind. Im Falle der Verwendung von derartigen Abmischungen von vorab hergestellten Diisocyanaten können bei der erfindungsgemäßen Verwendung selbstverständlich auch solche Gemische eingesetzt werden, die neben der immer erfindungswesentlichen Komponente a) der erfindungsgemäßen Gemische nur eine der Komponenten b) oder c) enthalten. Durch Abmischung der Einzelkomponenten können im übrigen auch solche erfindungsgemäße Diisocyanatgemische erhalten werden, die zwischen 50 und 100 Gew.-% der erfindungswesentlichen Komponente a) enthalten. Die erfindungsgemäßen Diisocyanate bzw. Diisocyanatgemische können selbstverständlich auch in Abmischung mit anderen an sich bekannten Polyisocyanaten der erfindungsgemäßen Verwendung zugeführt werden und als Ausgangsmaterialien zur Herstellung von Polyurethankunststoffen eingesetzt werden.

Die Herstellung von Polyurethanelastomeren erfolgt vorzugsweise unter Verwendung eines erfindungsgemäßen Diisocyanats (Komponente a)) oder unter Verwendung eines erfindungsgemäßen Diisocyanatgemischs (Komponenten a), b) und c)) durch deren Umsetzung mit

(i) di-und/oder trifunktionellen Polyhydroxylverbindungen des Molekulargewichtsbereichs 400 bis 10.000, vorzugsweise 800 bis 3.000, vorzugsweise den entsprechenden Polyhydroxypolyestern oder Polyhydroxypolyethern,

(ii) Kettenverlängerungsmittels des Molekulargewichtsbereichs 60 bis 399, d.h. mit im Sinne der Isocyanat-Additionsreaktion difunktionellen Verbindungen mit alkoholischen Hydroxylgruppen oder primären bzw. sekundären Aminogruppen, gegebenenfalls in Gegenwart von

(iii)weiteren aus der Chemie der Poluyrethanelastomeren an sich bekannten Hilfs- und Zusatzmitteln.

Die Umsetzung kann nach dem bekannten Prepolymerverfahren, Umsetzung der Diisocyanatkomponente mit der Komponente (i) unter einhaltung eines Äquivalentverhältnisses von Isocyanatgruppen zu gegenüber Isocyanatgruppen reaktionsfähigen Gruppen von größer als 1,3:1 und anschließende Umsetzung des so erhaltenen NCO-Prepolymeren mit der Komponente (ii) oder aber auch einstufig durch Umsetzung der Diisocyanatkomponente mit einem Gemisch der Komponenten (i) und (ii) erfolgen. Bei beiden Varianten liegt das Äquivalentverhältnis von Isocyanatgruppen zur Gesamtmenge der gegenüber Isocyanatgruppen reaktionsfähigen Gruppen im allgemeinen bei 0,8:1 bis 1,3:1, vorzugsweise 0,95:1 bis 1,1:1. Die Temperaturen, bei welchen diese Umsetzungen durchgeführt werden, liegen im allgemeinen bei 60 bis 180°C, vorzugsweise bei 80 bis 150°C. Die Umsetzungen können in Anwesenheit oder auch in Abwesenheit von geeeigneten inerten Lösungsmitteln erfolgen.

Bei den mit den erfindungsgemäßen Diisocyanaten (Komponente a)) oder Diisocyanatgemischen (Komponenten a), b) und c)) hergestellten Polyurethankunststoffen, insbesondere Polyurethanelastomeren kann es sich sowohl um massive als auch um zellförmige Produkte handeln. Die Herstellung beider Typen von Polyurethanelastomeren erfolgt nach den bekannten Verfahren, wie sie beispielsweise in der zuletzt genannten Literaturstelle beschrieben sind. So erfolgt beispielsweise die Herstellung von zellförmigen Polyurethanelastomeren unter Verwendung bzw. Mitverwendung von Wasser als Kettenverlängerungsmittel.

Die mit den erfindungsgemäßen Diisocyanaten (Komponente a)) oder Diisocyanatgemischen (Komponenten a), b) und c)) hergestellten Kunststoffe, insbesondere Elastomeren, besitzten hochwertige mechanische und thermische Eigenschaften. Sie sind deshalb hervorragend geeignet für Feder- und Dämpfungselemente, Puffer, Radbeläge, Dichtungen, Schuhsohlen und ähnliche Einsatzgebiete, bei denen das Material extremen mechanischen und thermischen Beanspruchungen ausgesetzt ist.

Die folgenden Beispiele sollen die Erfindungen näher erläutern. Prozentangaben beziehen sich auf Gewichtsprozente.

<u>Beispiel 1</u>

a) Herstellung von 4-Toluolsulfonsäure-[2-(4-nitrophenoxy)-ethyl]-ester

In einem 6 l-Kolben wurde eine Lösung von 915 g 2-(4-Nitrophenoxy)-ethanol in 1 l Chloroform vorgelegt und mit 950 g 4-Toluolsulfonsäurechlorid versetzt. Das Gemisch wurde unter Rühren auf 0° C gekühlt. In das Gemisch wurden im Verlauf von 3 Stunden unter Rühren und Kühlen 790 g Pyridin bei 0 bis 3° C eingetropft. Anschließend wurde das Reaktionsgemisch 2 Stunden bei Raumtemperatur nachgerührt und dann in eine Mischung von 4 kg Eis und 1,4 l konzentrierter Salzsäure eingerührt. Nach Zugabe von 2 l Chloroform wurde die organische Phase in einem Scheidetrichter abgetrennt, zweimal mit je 2 l Wasser gewaschen und im Vakuum vollständig eingeengt. Das zurückbleibende kristalline Rohprodukt wurde aus Essigester umkristallisiert und ergab 1466 g (87 % der Theorie) Kristalle vom Schmelzpunkt 121°C.
$C_{15}H_{15}NO_6S$ (337,4)

b) Herstellung von 1-(4-Nitrophenoxy)-2-(4-nitro-3-methyl-phenoxy)-ethan

In einem 10 l-Kolben wurden 6 l Ethylenglykol vorgelegt und unter Rühren mit 760 g einer 30%igen Lösung von Natriummethylat in Methanol und dann mit 612 g 4-Nitro-3-methyl-phenol versetzt. Aus dem Gemisch wurde dann bei 80° C Methanol abdestilliert, wobei der Druck so vermindert wurde, daß die Kopftemperatur 40 bis 50° C betrug. Anschließend wurde das Reaktionsgemisch bei 80° C portionsweise mit 1348 g 4-Toluolsulfonsäure-[2-(4-nitrophenoxy)-ethyl]-ester versetzt und dann 6 Stunden bei 100°C gerührt. Nach dem Abkühlen auf Raumtemperatur wurde das Gemisch in 14 l Wasser eingerührt. Die ausfallenden Kristalle wurden abgesaugt und zweimal mit je 1 l Wasser, dann zweimal mit je 1,5 l 5%iger Natronlauge, wiederum zweimal mit je 1 l Wasser und schließlich mit 1 l Methanol gewaschen. Nach dem Trocknen wurden die Kristalle aus Essigester umkristallisiert, wobei 1029 g (81 % der Theorie) Reinprodukt erhalten wurden.
$C_{15}H_{14}N_2O_6$ (318,3)
C ber. 56,6 %gef. 56,4 %
H ber. 4,4 %gef. 4,4 %
N ber. 8,8 %gef. 8,8 %

c) Herstellung von 1-(4-Aminophenoxy)-2-(4-amino-3-methyl-phenoxy)-ethan

In einem Hydrierautoklaven wurde eine Lösung von 1400 g 1-(4-Nitrophenoxy)-2-(4-nitro-3-methylphenoxy)-ethan in 7 l Dimethylformamid vorgelegt, mit Raney-Nickel versetzt und unter Rühren durch Aufdrücken von Wasserstoff umgesetzt. Die Hydrierung wurde bei 40 bis 50 bar 2 Stunden bei 75° C und dann 1 Stunde bei 90° C durchgeführt. Anschließend wurde der Autoklav entspannt, das heiße Reaktionsgemisch entnommen und der Katalysator durch Filtrieren abgetrennt. Die Reaktionslösung wurde

im Vakuum vollständig eingeengt, und das zurückbleibende Rohprodukt wurde zweimal aus Isopropanol umkristallisiert, wobei 1041 g (92 % der Theorie) Kristalle vom Schmelzpunkt 104° C erhalten wurden.
$C_{15}H_{18}N_2O_2$ (258,3)
C ber. 69,7 %gef. 69,2 %
H ber. 7,0 %gef. 7,0 %
N ber. 10,8 %gef. 10,5 %

d) Herstellung von 1-(4-Isocyanatophenoxy)-2-(4-isocyanato-3-methyl-phenoxy)-ethan

In einer 6 l-Laborphosgenierapparatur wurde eine Lösung von 350 g Phosgen in 3,8 l wasserfreiem Chlorbenzol vorgelegt und bei 0 bis 10° C mit 387 g 1-(4-Aminophenoxy)-2-(4-amino-3-methyl-phenoxy)-ethan versetzt. Unter Einleiten von zusätzlichem Phosgen (40 bis 50 g/h) wurde das Gemisch zügig auf 60°C, dann im Verlauf von 2,5 Stunden zum Rückfluß erhitzt. Nach einer weiteren Stunde Phosgenieren am Rückfluß war eine klare Lösung entstanden. Das Reaktionsgemisch wurde destillativ vom überschüssigen Phosgen und vom Lösungsmittel befreit. Das zurückbleibende Rohprodukt wurde durch Destillation im Vakuum gereinigt. Dabei destillierte das Diisocyanat bei 196 bis 198° C und 0,5 mbar ohne Vorlauf als farblose Flüssigkeit über, die rasch zu Kristallen vom Schmelzpunkt 86 bis 87°C erstarrte. Ausbeute: 446 g (96 % der Theorie)
$C_{17}H_{14}N_2O_4$ (310,3)
NCO-Gehalt: ber. 27,1 %gef.: 27,0 %
Das Diisocyanat ließ sich praktisch rückstandsfrei redestillieren und ergab farblose Kristalle mit unverändertem Schmelzpunkt und einem Gehalt an hydrolysierbarem Chlor von 70 ppm.

Beispiel 2 (Herstellung eines Gemischs von 1,2-Bis-(4-isocyanato-phenoxy)-ethan, 1-(4-Isocyanatophenoxy)-2-(4-isocyanato-3-methyl-phenoxy)-ethan und 1,2-Bis-(4-isocyanato-3-methyl-phenoxy)-ethan)

a) Herstellung eines Gemisches der entsprechenden Dinitroverbindungen

In einem mit einem Rückflußkühler versehenen 10 l-Kolben wurde ein Gemisch von 4,5 l Ethylenglykol, 1749 g wasserhaltigem 4-Nitro-3-methyl-phenol (67,0 %ig) und 1080 g wasserhaltigem 4-Nitrophenol (98,6 %ig) vorgelegt und unter Rühren mit 1226 g 50 %iger Natronlauge und 162 g wasserfreier Soda versetzt. Das Gemisch wurde im Waserstrahlvakuum bei 100 bis 110°C destillativ vom Wasser befreit und dann bei Normaldruck im Verlauf von etwa 3 Stunden bei 110 bis 120°C tropfenweise so mit 757 g 1,2-Dichlorethan versetzt, daß es schwach am Rück fluß siedete. Anschließend wurde das Reaktionsgemisch auf 130°C erhitzt, und weitere 152 g 1,2-Dichlorethan wurden im Verlauf von etwa einer Stunde bei 130 bis 140°C am schwachen Rückfluß eingetropft. Das Gemisch wurde 4 Stunden bei 140°C nachgerührt und dann auf Raumtemperatur abgekühlt. Das abgeschiedene Kristallisat wurde abgesaugt, in 5 l Wasser unter kräftigem Rühren kurz aufgekocht, nach dem Abkühlen wieder abgesaugt, mit 5 l Wasser und dann mit 1 l Methanol gewaschen und bei 70°C im Vakuum getrocknet:
2238 g braß-bräunliche Kristalle vom Schmelzbereich 169-177°C.

b) Herstellung eines Gemisches der entsprechenden Diamine

In einem Hydrierautoklaven wurden 6 l Dimethylformamid und 1900 g des im Beispiel 2a hergestellten Gemisches vorgelegt, mit Raney-Nickel versetzt und under Rühren durch Aufdrücken von Wasserstoff bei 40 bis 50 bar umgesetzt. Die Hydrierung wurde 2 Stunden bei 75°C und dann 1 Stunde bei 90°C durchgeführt. Anschließend wurde der Autoklav entspannt, das heiße Reaktionsgemisch entnommen und der Katalysator durch Filtrieren abgetrennt. Die Reaktionslösung wurde im Vakuum vollständig eingeengt, wobei 1448 g kristallines Produkt vom Schmelzbereich 81-85°C zurückblieben.

c) Herstellung des Diisocyanat-Gemisches

In einer 6 l-Laborphosgenierapparatur wurde eine Lösung von 350 g Phosgen in 3,8 l wasserfreiem Chlorbenzol vorgelegt und bei 0 bis 10°C mit 380 g des in Beispiel 2b) hergestellten Diamin-Gemisches versetzt. Unter Einleiten von zusätzlichem Phosgen (40 bis 50 g/h) wurde das Gemisch zügig auf 60°C, dann im Verlauf von 2,5 Stunden zum Rückfluß erhitzt. Nach einer weiteren Stunde Phosgenieren am Rückfluß war eine klare Lösung entstanden. Das Reaktionsgemisch wurde destillativ vom überschüssigen Phosgen und vom Lösungsmittel befreit. Das zurückbleibende Rohprodukt wurde durch Destillation im Vakuum gereinigt. Dabei destillierte das Diisocyanat-Gemisch bei 0,5 mbar und 194 bis 200°C ohne Vorlauf als farblose Flüssigkeit über, die langsam zu Kristallen erstarrte. Das Produkt wurde praktisch rückstandsfrei redestilliert, wobei 424 g farblose Kristalle vom Schmelzbereich 85-90°C erhalten wurden.
Zur Analyse wurde eine repräsentative Probe des Diisocyanat-Gemisches durch einstündiges Kochen in überschüssigem Ethanol zum entsprechenden Diethylurethan-Gemisch umgesetzt. Das Gemisch

wurde destillativ vollständig von Ethanol befreit und dann durch Hochdruckflüssigkeitschromatographie (HPLC) mit externem Standard analysiert. Durch Umrechnung ergab sich für das Diisocyanat-Gemisch folgende Zusammensetzung: 24,8 %, 1,2-Bis-(4-isocyanato-phenoxy)-ethan, 49,9 % 1-(4-Isocyanatophenoxy)-2-(4-isocyanato-3-methyl-phenoxy)-ethan und 25,2 % 1,2-Bis-(4-isocyanato-3-methyl-phenoxy)-ethan.
NCO-Gehalt des Diisocyanat-Gemisches:
ber. 27,1 %gef. 26,9 %.

Beispiel 3 (analog zu Beispiel 2)

a) Die Umsetzung wie im Beispiel 2a wurde wiederholt mit dem Unterschied, daß 1166 g 4-Nitro-3-methyl-phenol (67,0 %ig) und 1440 g 4-Nitrophenol (98,6 %ig) eingesetzt wurden. Es wurden 2165 g eines Gemisches von Dinitroverbindungen in Form bräunlicher Kristalle vom Schmelzbereich 150-155°C erhalten.

b) 1900 g des im Beispiel 3a hergestellten Gemisches wurden wie im Besipiel 2b) hydriert und aufgearbeitet. Dabei wurden 1438 g eines Diamin-Gemisches in Form von Kristallen vom Schmelzbereich 82-89°C erhalten.

c) Zur Herstellung des Diisocyanat-Gemisches wurden 380 g des im Beispiel 3b) hergestellten Diamin-Gemisches wie im Beispiel 2c) phosgeniert und aufgearbeitet. Bei der Destillatin des Rohproduktes destillierte das Gemisch ohne Vorlauf bei 0,5 mbar und 194 bis 203°C über. Die Redestillation ergab 419 g farblose Kristalle vom Schmelzbereich 76-80°C. Die Analytik des Gemisches wurde wie im Beispiel 2c) durchgeführt und ergab folgende Zusammensetzung: 43,1 % 1,2-Bis-(4-isocyanato-phenoxy)-ethan, 45,1 % 1-(4-Isocyanato-phenoxy)-2-(4-isocyanato-3-methyl-phenoxy)-ethan und 11,7 % 1,2-Bis-(4-isocyanato-3-methyl-phenoxy)-ethan.
NCO-Gehalt des Diisocyanat-Gemisches:
ber. 27,5 %gef. 27,2%

Beispiel 4(analog zu Beispiel 2)

a) Die Umsetzung wie im Beispiel 2a) wurde wiederholt mit dem Unterschied, daß 2332 g 4-Nitro-3-methyl-phenol (67,0 %ig) und 720 g 4-Nitrophenol (98,6 %ig) eingesetzt wurden. Es wurden 2279 g eines Gemisches von Dinitroverbindungen im Form blaß-bräunlicher Kristalle vom Schmelzbereich 175-178°C erhalten.

b) 1900 g des im Beispiel 4a) hergestellten Gemisches wurden wie im Beispiel 2b) hydriert und aufgearbeitet. Dabei wurden 1454 g eines Diamin-Gemisches in Form von Kristallen vom Schmelzbereich 87-90°C erhalten.

c) Zur Herstellung des Diisocyanat-Gemisches wurden 380 g des im Beispiel 4b) hergestellten Diami-Gemisches wie im Beispiel 2c) phosgeniert und aufgearbeitet. Bei der Destillation des Rohproduktes destillierte das Gemisch ohne Vorlauf bei 0,4 mbar und 195 bis 199°C über. Die Redestillation ergab 422 g farblose Kristalle vom Schmelzbereich 90-100°C. Die Analytik des Gemisches wurde wie im Beispiel 2c) durchgeführt und ergabe folgende Zusammensetzung: 10,3 % 1,2-Bis-(4-isocyanato-phenoxy)-ethan, 42,5 % 1-(4-Isocyanato-phenoxy)-2-(4-isocyanato-3-methyl-phenoxy)-ethan und 47,0 % 1,2-Bis-(4-isocyanato-3-methyl-phenoxy)-ethan. NCO-Gehalt des Diisocyanat-Gemisches:
ber. 26,6 %gef. 26,6 %.

Beispiel 5 (Herstellung eines Gemisches von 1,4-Bis-(4-isocyanato-phenoxy)-butan, 1-(4-Isocyanato-phenoxy)-4-(4-isocyanato-3-methyl-phenoxy)-butan und 1,4-Bis-(4-isocyanato-3-methyl-phenoxy)-butan)

a) Die Umsetzung wie im Beispiel 2a) wurde wiederholt mit dem Unterschied, daß anstelle von 1,2-Dichlorethan 1,4-Dichlorbutan (1. Portion: 972 g; 2. Portion: 98 g) eingesetzt wurde und die Umsetzung ohne Rückfluß verlief. Es wurden 2306 g eines Gemisches von Dinitroverbindungen in Form bräunlicher Kristalle vom Schmelzbereich 88-94°C erhalten.

b) 1900 g des im Beispiel 5a) hergestellten Gemisches wurden wie im Beispiel 2b) hydriert und aufgearbeitet. Nach dem Abziehen dez Lösungsmittels blieben 1417 g eines Diamin-Gemisches in Form einer bräunlichen, viskosen Flüssigkeit zurück.

c) Zur Herstellung des Diisocyanat-Gemisches wurden 420 g des im Beispiel 5b) hergestellten Diamin-Gemisches wie im Beispiel 2c) phosgeniert und aufgearbeitet. Bei der Destillation des Rohproduktes destillierte das Gemisch ohne Vorlauf bei 0,2 mbar und 174 bis 178°C über. Die Redestillation ergab 413 g einer farblosen Flüssigkeit, die nur langsam zu Kristallen vom Schmelzbereich 62-69°C erstarrte. Die Analytik des Gemisches wurde wie im Beispiel 2c) durchgeführt und ergab folgende Zusammensetzung; 25,5 % 1,4-Bis-(4-isocyanato-phenoxy)-butan, 46,3 % 1-(4-Isocyanato-phenoxy)-4-(4-isocyanato-3-methyl-phenoxy)-butan und 28,1 % 1,4-Bis-(4-isocyanato-3-methyl-phenoxy)-butan.
NCO-Gehalt des Diisocyanat-Gemisches:
ber. 24,8 %gef. 25,1 %

## Patentansprüche

1. Diisocyanate oder Diisocyanatgemische, dadurch gekennzeichnet, daß sie
a) zu 30 bis 100 Gew.-% aus Diisocyanaten der allgemeinen Formel

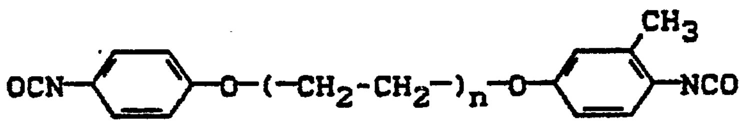

,

b) zu 0 bis 50 Gew.-% aus Diisocyanaten der allgemeinen Formel

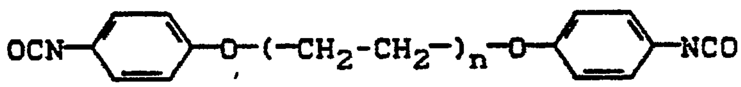

und
c) zu 0 bis 50 Gew.-% aus Diisocyanaten der allgemeinen Formel

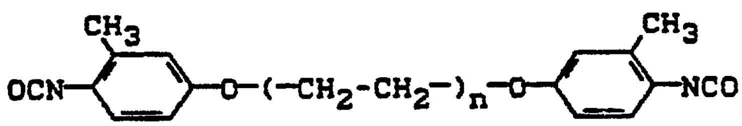

bestehen, wobei

n in diesen Formeln die Bedeutung von 1, 2 oder 3 hat,
und wobei sich die genannten Prozentsätze zu 100 ergänzen.

2. Diisocyanatgemische gemäß Anspruch 1, dadurch gekennzeichnet, daß die Komponente a) in einer Menge von 30 bis 50 Gew.-%, die Komponente b) in einer Menge von 5 bis 45 Gew.-% und die Komponente c) in einer Menge von 5 bis 45 Gew.-% vorliegen, wobei sich die genannten Prozentsätze zu 100 ergänzen.

3. Diisocyanatgemische gemäß Anspruch 1 und 2, dadurch gekennzeichnet, daß die Komponente a) in einer Menge von 35 bis 50 Gew.-%, die Komponente b) in einer Menge von 10 bis 35 Gew.-% und die Komponente c) in einer Menge von 10 bis 35 Gew.-% vorliegen, wobei sich die genannten Prozentsätze zu 100 ergänzen.

4. Verfahren zur Herstellung von Diisocyanaten oder von Diisocyanatgemischen gemäß Anspruch 1 bis 3, dadurch gekennzeichnet, daß man
a) Diamine der allgemeinen Formel

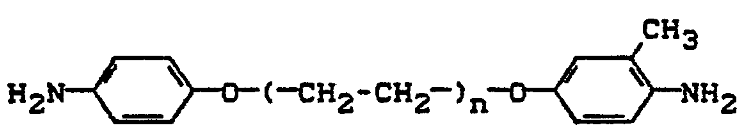

oder Gemische von diesen Diaminen mit
b) Diaminen der allgemeinen Formel

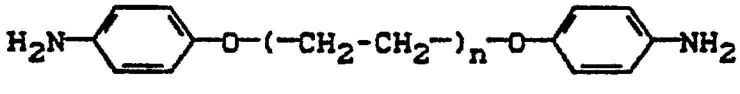

und mit
c) Diaminen der allgemeinen Formel

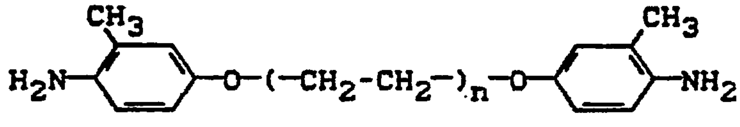

oder deren Addukte mit Chlorwasserstoff oder Kohlendioxid in an sich bekannter Weise mit Phosgen umsetzt, wobei n in diesen Formeln die in Anspruch 1 genannte Bedeutung hat, und wobei die mengenmäßige Zusammensetzung der gegebenenfalls eingesetzten Diamingemische so gewählt wird, daß Diisocyanatgemische der in Anspruch 1 bis 3 genannten Zusammensetzung resultieren.

5. Verwendung der Diisocyanate bzw. Diisocyanatgemische gemäß Anspruch 1 bis 3 als Aufbaukomponente bei der Herstellung von Polyurethankunststoffen nach dem Isocyanat-Polyadditionsverfahren.

## Claims

1. Diisocyanates or diisocyanate mixtures, characterized in that

a) from 30 to 100% by weight thereof consists of diisocyanates corresponding to the following general formula

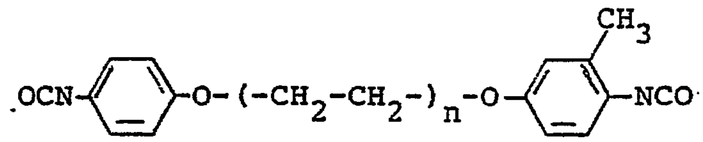

b) from 0 to 50% by weight of diisocyanate corresponding to the following general formula

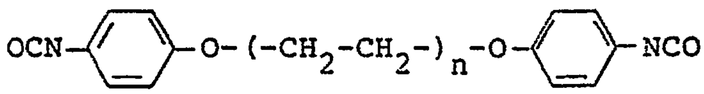

and

c) from 0 to 50% by weight of diisocyanates corresponding to the following general formula

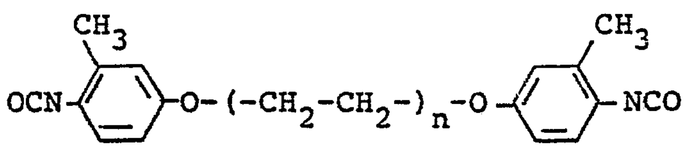

n in these formulae having a value of 1, 2 or 3 and the percentages indicated adding up to 100.

2. Diisocyanate mixtures as claimed in Claim 1, characterized in that component a) is present in a quantity of from 30 to 50% by weight, component b) in a quantity of from 5 to 45% by weight and component c) in a quantity of from 5 to 45% by weight, the percentages indicated adding up to 100.

3. Diisocyanate mixtures as claimed in Claims 1 and 2, characterized in that component a) is present in a quantity of from 35 to 50% by weight, component b) in a quantity of from 10 to 35% by weight and component c) in a quantity of from 10 to 35% by weight, the percentages indicated adding up to 100%.

4. A process for producing the diisocyanates or diisocyanate mixtures claimed in Claims 1 to 3, characterized in that

a) diamines corresponding to the following general formula

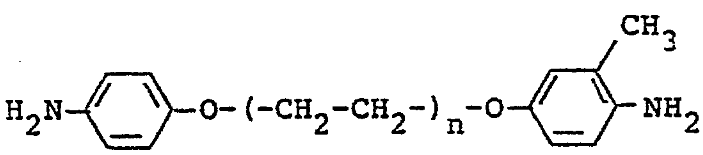

or mixtures thereof with

b) diamines corresponding to the following general formula

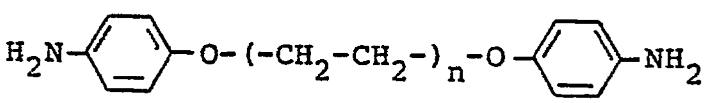

and with

c) diamines corresponding to the following general formula

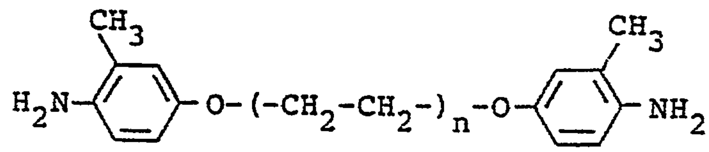

or adducts thereof with hydrogen chloride or carbon dioxide are reacted in known manner with phosgene, n in the above formulae having the meaning defined in Claim 1 and the quantitative composition of the diamine mixtures optionally used being selected so that diisocyanate mixtures having the composition defined in Claims 1 to 3 are obtained.

5. The use of the diisocyanates or diisocyanate mixtures claimed in Claims 1 to 3 as synthesis component in the production of polyurethane plastics by the isocyanate polyaddition process.

**Revendications**

1. Diisocyanates ou mélanges de diisocyanates, caractérisés en ce qu'ils consistent en:
a) 30 à 100% en poids de diisocyanates de formule générale

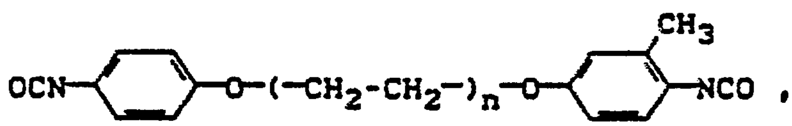

b) 0 à 50% en poids de diisocyanates de formule générale

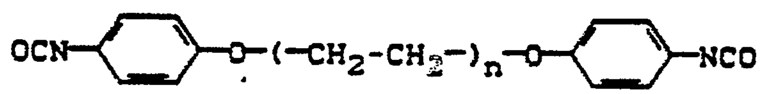

et
c) 0 à 50% en poids de diisocyanates de formule générale

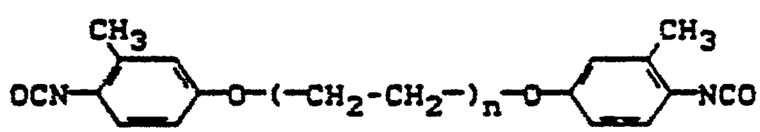

n, dans ces formules, étant égal à 1, 2 ou 3, et les pourcentages indiqués se complétant à 100%.

2. Mélanges de diisocyanates selon la revendication 1, caractérisés en ce que le composant a) est présent en quantités de 30 à 50% en poids, le composant b) en quantités de 5 à 45% en poids et le composant c) en quantités de 5 à 45% en poids, ces pourcentages se complétant à 100%.

3. Mélanges de diisocyanates selon les revendications 1 et 2, caractérisés en ce que le composant a) est présent en quantités de 35 à 50% en poids, le composant b) en quantités de 10 à 35% en poids et le composant c) en quantités de 10 à 35% en poids, ces pourcentages se complétant mutuellement à 100%.

4. Procédé de préparation des diisocyanates ou mélanges de diisocyanates selon les revendications 1 à 3, caractérisé en ce que l'on fait réagir avec du phosgène, de manière connue en soi:
a) des diamines de formule générale

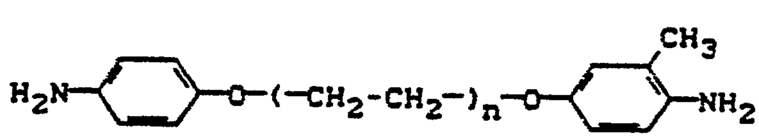

ou des mélanges de ces diamines avec
b) des diamines de formule générale

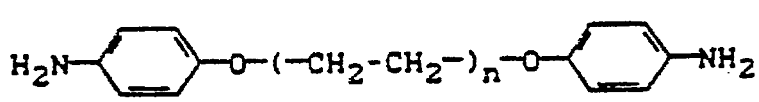

et
c) des diamines de formule générale

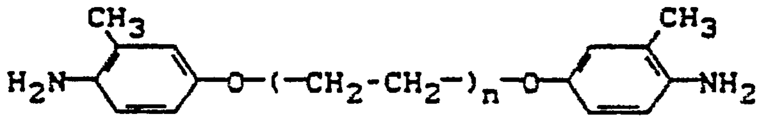

ou leurs adducts avec le chlorure d'hydrogène ou le dioxyde de carbone,
n ayant dans ces formules les significations indiquées dans la revendication 1, et la composition en poids de mélanges de diamines éventuellement mis en œuvre étant choisie de manière à obtenir des mélanges de diisocyanates à la composition indiquée dans les revendications 1 à 3.

5. Utilisation des diisocyanates et mélanges de diisocyanates selon les revendications 1 à 3 en tant que composants de synthèse à la préparation de résines synthétiques de polyuréthannes par le procédé de polyaddition des isocyanates.